# EUROPEAN PATENT SPECIFICATION

(11) **EP 3 035 919 B1**
(45) Date of publication and mention of the grant of the patent: **09.10.2019**
(21) Application number: 14790300.9
(22) Date of filing: 18.08.2014
(51) Int. Cl.: A61K 9/28, A61K 31/381, A61K 9/20

(54) **DULOXETINE ENTERIC COATED TABLET**
DARMLÖSLICHE DULOXETIN-BESCHICHTETE TABLETTE
COMPRIMÉ DE DULOXÉTINE À ENROBAGE ENTÉRIQUE

(30) Priority: 21.08.2013 PL 40511113
(43) Date of publication of application: 29.06.2016
(73) Proprietor: Adamed Pharma Spólka Akcyjna, 05-152 Czosnów (PL)
(72) Inventor: TRELA, Jolanta, 95-200 Pabianice (PL); KOWALSKA, Ewelina, 95-200 Pabianice (PL)
(74) Representative: Sitkowska, Jadwiga
(86) International application number: PCT/IB2014/063955
(87) International publication number: WO 2015/025261

(56) References cited:
- EP-A1- 2 380 563
- "Sugar, Compressible" In: "Handbook of Pharmaceutical Excipients - Fifth Edition", 2006, Pharmaceutical Press, UK / USA, XP055156899, ISBN: 978-0-85-369618-6 pages 748-749, the whole document
- RAJA SUBBURAYALU ET AL: "Formulation and Stabilization of Duloxetine Hydrochloride Delayed Release Pellets with the Aid Non Ionic Barrier Layer", IOSR JOURNAL OF PHARMACY, vol. 03, no. 07, 1 January 2013 (2013-01-01), pages 63-70, IN ISSN: 2319-4219, DOI: 10.9790/3013-0371063-70

## Description

The present invention is directed to an enteric-coated tablet comprising duloxetine hydrochloride and a process for the preparation of said enteric-coated tablet.

Duloxetine, (S)-(+)-N-methyl-3-(1-naphthalenyloxy)-2-tiophenpropylamine is the inhibitor of serotonin (5-HT) and noradrenaline (NA) reuptake. This compound and its properties are disclosed in EP0273658A. Duloxetine is used in the treatment of major depressive disorders, treatment of peripheral diabetic neuropathy, treatment of generalized anxiety disorders, as well as in the treatment of moderate to severe stress urinary incontinence in women. In the commercially available medicinal preparations, e.g. Cymbalta® and Yentreve®, duloxetine is present in the form of hydrochloride salt.

Due to the instability of duloxetine in acidic environment (among others due to acid catalyzed hydrolysis with formation of toxic 1-naphthol), duloxetine is administered orally in the form of enteric coated compositions. However, as demonstrated by the first studies on this type of preparations, duloxetine reacts with many ingredients of enteric coatings, which include polymers with acidic groups, to form slowly dissolving or even insoluble layers. This results in unsatisfactory dissolution profile and low bioavailability. The solution to the above problem were the pellets as disclosed in EP0693282A, in which between the duloxetine-containing core and the coating including hydroxypropylmethylcellulose acetate succinate (HPMCAS) there was provided a separating layer (coating), which prevented direct contact between duloxetine and the polymer of the enteric coating.

Prior art describes many enteric compositions of duloxetine, in which duloxetine is usually separated by a layer insulating from the enteric coating. Such compositions are described, among others, in WO2007/139886, WO2008/020286, EP1938840A, WO2009/066181, WO2009/001043, WO2009/010238 and WO2010/037849. In some compositions, among others described in EP2133072A, WO2009/087657 and WO2009/150238, in addition to the separating layer, further duloxetine-stabilizing substances were used in order to reduce the adverse impact of the acidic environment, including the effect of the acidic groups of the polymers present in enteric coatings.

WO2004/047830 describes a duloxetine comprising tablet composition, wherein the tablet was directly coated with a coating comprising Eudragit L-30 D55 (methacrylic acid and ethyl acrylate copolymer 1:1). However, as found by the present inventors, the composition of the core was not adequate to ensure appropriate stability and release profile of duloxetine. The impurities levels increased very fast, among others due to the applied wet granulation method as well as due to the presence of lactose and developed contact surface area of the enteric polymer.

Double coating of duloxetine composition increases costs and complicates the manufacturing process, and not always results in achievement of desired effect, especially in the context of duloxetine stability and appropriate release profile. As noted in WO2007/139886, a mixture of duloxetine coating composition comprising hydroxypropylmethylcellulose acetate succinate (HPMCAS), as well as tube and nozzle of the coater, should be cooled prior to application. Furthermore, as described in EP0693282A, HPMCAS is subjected to neutralization with ammonia in order to facilitate dissolution.

Therefore, when it comes to ease of preparation and coating technology, methacrylic acid copolymers and hydroxypropylmethylcellulose phthalate have numerous advantages as an enteric coating polymers compared to HPMCAS. For example, they can be applied at ambient temperatures using conventional machinery and do not require neutralization with bases. However, P. J. Jansen et al. (J. Pharm. Sci., Vol 87 (1) p. 81-85 (1998)), in the context of hydroxypropylmethylcellulose phthalate, described the phenomenon of the increase of duloxetine degradation products, resulting from the migration of the constituents of enteric coating composition through the separating layer or by damages of this layer. Increasing the thickness of the separating layer was proposed as a solution to this problem.

EP2380563A discloses an enteric formulation of duloxetine comprising a core comprising duloxetine and water soluble hot melt material, a separating layer, and an enteric layer.

There is therefore a need for a stable composition of duloxetine with a suitable release profile, which is obtainable in a simple, economical process.

It has been surprisingly found that stable enteric-coated compositions of duloxetine with bioavailability and release profile corresponding to the commercially available duloxetine compositions, can be prepared without exposing duloxetine to the contact with granulation liquids and without application of a separating coating. The present inventors have found, that it is possible to obtain enteric-coated tablets of duloxetine, wherein the core is directly coated with a single enteric coating and wherein the active ingredient remains stable throughout the period of storage, and which can be prepared in a simple and economical process.

The present invention provides an enteric-coated tablet of duloxetine consisting of:
- a core comprising duloxetine hydrochloride, which is blended uniformly without the use of any liquid with compressible sugar as defined in the monograph of the British Pharmacopoeia 2013, Volume II, pages 2117-2118, the amount of said compressible sugar being 60-85% by weight calculated on the total weight of the core, and optionally with one or more pharmaceutically acceptable excipients, and
- an enteric coating applied directly on the core and comprising methacrylic acid-ethyl acrylate copolymer (1:1) as the only polymer resistant to gastric acid and one or more pharmaceutically acceptable excipients.

In a preferred embodiment, the percentage of the compressible sugar is 65-70% by weight, and still more preferably 67% by weight calculated on the total weight of the core.

The compressible sugar, also described in the art as a directly compressible sugar or direct compacting and tableting sugar, in the context of this invention means a substance as defined in the monograph of the British Pharmacopoeia 2013, Volume II, pages 2117-2118. The compressible sugar is defined here as containing sucrose (95-98% by weight) and maltodextrin or dry glucose syrup (2-5% by weight). It may further comprise a suitable lubricant, invert sugar or suitable coloring agent. The compressible sugar is in the form of dry free-flowing powder or microcrystalline agglomerates.

In a preferred embodiment of the enteric-coated tablet of the invention the compressible sugar is a spray-dried mixture of sucrose, invert sugar and maltodextrins, wherein the percentage of sucrose is 95-98% by weight, and the percentage of invert sugar and maltodextrins amounts in total 2-5% by weight, calculated on the total weight of the compressible sugar. An example of a commercially available compressible sugar is Compressuc MS.

In a further embodiment of the enteric-coated duloxetine tablet according to the invention, the percentage of duloxetine hydrochloride is 15-30% by weight, and preferably 23% by weight, calculated on the total weight of the core.

Duloxetine enteric-coated tablet of the invention may further comprise in the core one or more pharmaceutically acceptable excipients, preferably selected from the group consisting of a glidant, lubricant, and mixtures thereof.

In a preferred variant, the core of the duloxetine enteric-coated tablet in addition to the active ingredient and the compressible sugar comprises a mixture of a glidant and lubricant.

In a preferred embodiment the percentage of the glidant is 7-11% by weight, preferably 9% by weight, and the percentage of lubricant is 0.5-3% by weight, preferably from 1 to 0.5% by weight, most preferably 0.5% by weight, calculated on the total weight of the core.

Preferably, the glidant is corn starch, and the lubricant is magnesium stearate.

Preferably, the enteric coating of the tablet according to the invention comprises methacrylic acid and ethyl acrylate copolymer (1:1) in the amount of 60-70% by weight, more preferably 67% by weight, calculated on the total weight of the enteric coating.

Surprisingly, the inventors have found that in the case of the enteric coating according to the invention there is no need to use basic substances neutralizing free carboxyl groups of the methacrylic acid-ethyl acrylate copolymer.

Examples of commercially available methacrylic acid-ethyl acrylate copolymers (1:1) are EUDRAGIT® L 100-55 in the form of a solid substance, and Eudragit ® L 30 D-55, which is in the form of ready to use 30% aqueous dispersion of said copolymer.

The enteric-coated tablet according to the present invention must ensure the passage of the drug through the stomach of a patient substantially without release of the active substance. The active substance comprised in such tablets should be released and dissolved only in the small intestine of the patient, and this is provided by the enteric coating which is insoluble in the acidic environment of the gastric juice.

One or more pharmaceutically acceptable excipients present in the enteric coating is preferably selected from the group consisting of a plasticizer, anti-caking agent, pigment, dye, substance preventing foaming of the coating suspension during the coating process, and mixtures thereof.

In a preferred embodiment of the invention one or more excipients in the enteric coating is in the form of a mixture of a plasticizer, anti-caking agent and pigment.

In a preferred embodiment of the invention, the percentage of the plasticizer is 8-12% by weight, preferably 10% by weight, the percentage of the anti-caking agent is 18-22% by weight, preferably 20% by weigh, and the percentage of the pigment is 2-5% by weight, preferably 3% by weight, calculated on the total weight of the enteric coating.

Preferably, the plasticizer is triethyl citrate, the anti-caking agent is talc, and the pigment is titanium dioxide.

The percentage of the enteric coating preferably is 8-13% by weight, more preferably 9-10% by weight, calculated on the total weight of the enteric-coated tablet of the invention.

The tablet according to the invention comprises duloxetine hydrochloride in the amount equivalent to 20, 30, 40 or 60 mg of duloxetine free base. At these doses, duloxetine is currently used in the treatment of depression and diabetic neuropathy (30 and 60 mg), and the treatment of stress urinary incontinence (20 and 40 mg).

Another object of the invention is a process for the preparation of an enteric-coated tablets, as described above, comprising the steps of:
a) preparing a blend of duloxetine hydrochloride with compressible sugar and optionally one or more pharmaceutically acceptable excipients, wherein the blend is prepared without the use of any liquid,
b) tableting the resulting blend to form tablet cores, and
c) coating the tablet cores with enteric coating material comprising methacrylic acid-ethyl acrylate copolymer (1:1) and one or more pharmaceutically acceptable excipients, wherein said coating is applied directly onto the tablet core.

The blend prepared in step a) and tableted in step b) can be a simple blend.

The blend prepared in step a) can be also a granulate prepared by dry-granulation or a mixture of said granulate with a lubricant.

Accordingly, the blend is prepared in step a) by blending duloxetine hydrochloride with compressible sugar and optionally one or more pharmaceutically acceptable excipients, wherein the blend is prepared without the use of any liquid.

Preferably, the blend is prepared in step a) by blending duloxetine hydrochloride with compressible sugar and a glidant.

In a preferred embodiment of the above process, in step a) duloxetine hydrochloride is blended with compressible sugar and optionally one or more pharmaceutically acceptable excipients and the resulting blend is further subjected to dry granulation before tableting in step b).

In a more preferred variant of the above embodiment of the present invention duloxetine hydrochloride is blended with compressible sugar and a glidant, preferably corn starch, the resulting blend is further subjected to dry granulation, then the resulting granulate is mixed with a lubricant, preferably magnesium stearate and thus obtained blend is subjected to tableting step b).

Direct application of the enteric coating in the context of the present invention means that the tablet core is in direct contact with the entire surface of the enteric coating; thus, in-between the core and the enteric coating there is no separating coating (layer).

According to the invention, the term "blended uniformly" used to define the core of the tablet means that duloxetine hydrochloride, compressible sugar and optionally one or more pharmaceutically acceptable excipients means that said components of the core are subjected to stirring without any liquids (e.g. solvents) and that in samples being a part of mass or volume of the resulting blend the proportions (percentages/shares) of the components are substantially the same.

Manufacturing and coating of the tablets according to the invention are performed according to technological operations known in the pharmaceutical technology which can be realized using standard methods and means known in the art and described for example in the "Farmacja stosowana" edited by S. Janicki, A. Fiebig, PZWL, ed. 4.

Blending operations can be performed in conventional blenders used in the pharmaceutical dosage forms technology.

Dry granulation, i.e. granulation without the use of any liquid can be carried out using standard equipment. In a preferred embodiment of the process according to the invention, granulation without the use of any liquids, is carried out by compacting or briquetting. Preferably, compacting is carried out with the use of a roller compactor.

Tableting to form the cores can be carried out using a rotary tablet press.

Coating of the tablet cores with the enteric coating according to the invention can be performed using a pan coater with perforated drum or using a fluidized bed coater.

The invention and disclosure will be further described in the following examples, which do not limit the scope of the invention.

### Example 1.

Four batches of enteric-coated tablets of duloxetine of various doses were prepared according to the process of the invention as described below. The amounts of components for each duloxetine dose are presented in Table 1 below.

**Table 1**

| Component | duloxetine dose [mg] in a tablet (calculated on duloxetine free base) | | | |
|---|---|---|---|---|
| | 20 | 30 | 40 | 60 |
| *Components of the tablet core [% by weight, calculated on total weight of the core]* | | | | |
| Duloxetine hydrochloride | 23 | 23 | 23 | 23 |
| Compressible sugar (Compressuc MS) | 67 | 67 | 67 | 67 |
| Corn starch | 9 | 9 | 9 | 9 |
| Magnesium stearate | 1 | 1 | 1 | 1 |

| *Components of the enteric coating [% by weight, calculated on the total weight of the coating]* | | | | |
|---|---|---|---|---|
| methacrylic acid and ethyl acrylate copolymer (1:1) | 67 | 67 | 67 | 67 |
| Triethyl citrate | 10 | 10 | 10 | 10 |
| Talk | 20 | 20 | 20 | 20 |
| Titanium dioxide | 3 | 3 | 3 | 3 |
| percentage of the enteric coating *[% by weight, calculated on the total mass of the tablet]* | 10 | 10 | 9 | 9 |
| **Total mass of the tablet [mg]** | 111.0 | 166.5 | 220.0 | 330.0 |

| | | | | |
|---|---|---|---|---|
| *as a dry mass; 30% aqueous dispersion of methacrylic acid-ethyl acrylate copolymer (1:1) (Eudragit ® L30 D-55) was used for coating | | | | |

Duloxetine hydrochloride, compressible sugar and corn starch were blended vigorously for 20 minutes using high shear mixer-granulator DIOSNA. The resulting blend was compacted using roll compactor Fitzpatrick, at compactor rolls contact pressure of 60 bar, and using refining grid with 1 mm mesh. The resulting granulate was mixed with magnesium stearate in Pharmatech mixer (stirring time: 5 minutes), and then tableted using rotary tablet press Fette. Thus obtained tablet cores were coated by means of a pan coater with perforated drum using a suspension prepared by mixing 30% aqueous dispersion of methacrylic acid and ethyl acrylate copolymer (1:1) with the other ingredients of the coating (as specified in Table 1).

**Example 2.** Two batches of duloxetine enteric-coated tablets of various doses were prepared analogously to the process of the invention described in Example 1. The amounts of components for each duloxetine dose are presented in Table 2 below:

**Table 2**

| Component | duloxetine dose [mg] in a tablet (calculated on duloxetine free base) | | | |
|---|---|---|---|---|
| | 20 | 30 | 40 | 60 |
| *Components of the tablet core [% by weight, calculated on total weight of the core]* | | | | |
| Duloxetine hydrochloride | 23 | 23 | 23 | 23 |
| Compressible sugar (Compressuc MS) | 67 | 67 | 67 | 67 |
| Corn starch | 9.5 | 9.5 | 9.5 | 9.5 |
| Magnesium stearate | 0.5 | 0.5 | 0.5 | 0.5 |

| *Components of the enteric coating [% by weight, calculated on the total mass of the coating]* | | | | |
|---|---|---|---|---|
| methacrylic acid and ethyl acrylate copolymer (1:1) | 67 | 67 | 67 | 67 |
| Triethyl citrate | 10 | 10 | 10 | 10 |
| Talk | 20 | 20 | 20 | 20 |
| Titanium dioxide | 2.85 | 2.85 | 2.85 | 2.85 |
| percentage of the enteric coating *[% by weight, calculated on the total mass of the tablet]* | 9 | 9 | 8 | 8 |
| **Total mass of the tablet [mg]** | 108.5 | 162.68 | 214.92 | 322.38 |

| | | | | |
|---|---|---|---|---|
| *as a dry mass; 30% aqueous dispersion of methacrylic acid-ethyl acrylate copolymer 1:1 (Eudragit ® L30 D-55) was used for coating | | | | |

### Example 3.

Stability study obtained for tablets 60 mg (tablet composition as described in Example 1). The content of impurities was determined by HPLC. Conditions of the test and results are presented in the Table 3 below.

**Table 3**

| | **impurities content** | |
|---|---|---|
| conditions | start | 1 month |
| 25°C/60% RH | 0.07% | 0.09% |
| 30°C/60% RH | 0.07% | 0.11% |
| 40°C/75% RH | 0.07% | 0.14% |

### Example 4.

Stability study obtained for tablets 60 mg (tablet composition as described in Example 2). The content of impurities was determined by HPLC. Conditions of the test and results are presented in the Table 4 below.

**Table 4**

| | **impurities content** | | | |
|---|---|---|---|---|
| conditions | start | 3 months | 6 months | 12 months |
| 25°C/60% RH | 0.1% | 0.1% | 0.2% | 0.1% |
| 30°C/60% RH | 0.1% | 0.1% | 0.1% | 0.2% |
| 40°C/75% RH | 0.1% | 0.1% | 0.2% | |

Enteric-coated tablets obtained according to Example 1 and Example 2 were also tested in vitro and the release profile was determined using a similarity factor method according to the guideline for modified release oral dosage forms CPMP/QWP/604/96. For example, in the similarity test enteric-coated tablets 30 and 60 mg according to the invention show the similarity factor f2 equal to 69 and 74, respectively, compared to Cymbalta profile 30 and 60 mg as a reference profile. According to the above cited guideline, f2 value above 50 indicates similarity of the profiles.

By the use of compressible sugar as a filler, the present inventors have surprisingly managed to obtain duloxetine tablet cores with very specific and advantageous properties of the active substance. The tablet cores during the release and breakdown tests behaved in a manner described by those skilled in the art as the "leaching out" of the tablets. This phenomenon is based on the gradual elution of the surface of tablets in a release assay. In the case of duloxetine, tablet core with such a property has a very advantageous characteristics, providing protection against too rapid disintegration of the tablet, and preventing from the contact of too large surface of duloxetine with the polymer of enteric coating, which would result in the increase of impurities levels.

Despite the presence of acidic groups in the methacrylic acid-ethyl acrylate copolymer (1:1) and their contact with duloxetine hydrochloride present at the surface of the tablet cores according to the invention, the negative phenomena (formation of sparingly soluble layers, significant increase of impurities) are not observed in the case of the duloxetine tablets of the invention. Therefore, there is surprisingly no need for use of a separation layer, which results in considerable simplification of the preparation process of the duloxetine tablet.

Enteric coatings, although not dissolving in the stomach, are wetted during maceration in the acidic environment. In the case of a tablet core which has the characteristics of swelling and disintegration into small particles (granules or powder), as for example in the case of the core known from WO2004/047830, wetting of the enteric coating increases the risk of degradation of the active substance sensitive to acid environment, comparing to the so-called "leaching-out" core according to the invention. This is because the structure of wetted core undergoes loosening more easily and absorbs the moisture from the gastric juice, which because of pH value would degrade duloxetine.

As it was found for the tablets of the present invention, the composition of the core based on compressible sugar is a factor that enhances protection of duloxetine against the degrading effect of acidic environment. Furthermore, the form of duloxetine tablet is here much more favorable than, for example, granules or pellets, because in the absence of a separating layer the contact surface with the enteric coating polymer is the lowest possible. In contrast to the coatings based on hydroxypropylmethylcellulose phthalate, in the tablets of the invention methacrylic acid-ethyl acrylate copolymer despite the presence of unneutralized acid groups does not lead to the increase of impurities level beyond the accepted limits.

Additionally, duloxetine tablets according to the invention exhibit improved flowability properties. Appropriate concentration of the lubricant, preferably magnesium stearate, present in the tablet according to the present invention decreases the stickiness and agglomeration of the blend during the manufacturing process. The inventors found that the amount of preferred magnesium stearate lubricant in the duloxetine tablet core is preferably in the range of 1 to 0.5% by weight, calculated on total weight of the core, most preferably 0.5% by weight, calculated on total weight of the core.

## Claims

1. Enteric-coated duloxetine tablet consisting of:
- a core comprising duloxetine hydrochloride, which is blended uniformly without the use of any liquid with compressible sugar as defined in the monograph of the British Pharmacopoeia 2013, Volume II, pages 2117-2118, the amount of said compressible sugar being 60-85% by weight calculated on the total weight of the core, and optionally with one or more pharmaceutically acceptable excipients, and
- an enteric coating applied directly on the core and comprising methacrylic acid-ethyl acrylate copolymer (1:1) as the only polymer resistant to gastric acid and one or more pharmaceutically acceptable excipients.

2. Enteric-coated duloxetine tablet according to claim 1, **characterized in that** the compressible sugar is a spray-dried mixture of sucrose, invert sugar and maltodextrins, wherein the percentage of sucrose is 95-98% by weight, and the percentage of invert sugar and maltodextrins amounts in total 2-5% by weight, calculated on the total weight of the compressible sugar.

3. Enteric-coated duloxetine tablet according to claims 1 or 2, **characterized in that** the percentage of the compressible sugar is 65-70% by weight, preferably 67% by weight calculated on the total weight of the core.

4. Enteric-coated duloxetine tablet according to any of the claims 1 to 3, **characterized in that** the percentage of duloxetine hydrochloride is 15-30% by weight, preferably 23% by weight, calculated on the total weight of the core.

5. Enteric-coated duloxetine tablet according to any of the claims 1 to 4, **characterized in that** one or more pharmaceutically acceptable excipients in the core is preferably selected from the group consisting of a glidant, lubricant, and mixtures thereof.

6. Enteric-coated duloxetine tablet according to claim 5, **characterized in that** one or more pharmaceutically acceptable excipients in the core is a mixture of the glidant in the amount of 7-11% by weight, preferably 9% by weight and the lubricant in the amount of 0.5-3% by weight, preferably from 1 to 0.5% by weight, most preferably 0.5% by weight, calculated on the total weight of the core.

7. Enteric-coated duloxetine tablet according to claim 6, **characterized in that** the glidant is corn starch and the lubricant is magnesium stearate

8. Enteric-coated duloxetine tablet according to any of the claims 1 to 7, **characterized in that** the percentage of methacrylic acid and ethyl acrylate copolymer (1:1) is 60-70% by weight, more preferably 67% by weight, calculated on the total weight of the enteric coating.

9. Enteric-coated duloxetine tablet according to any of the claims 1 to 8, **characterized in that** one or more pharmaceutically acceptable excipients present in the enteric coating is selected from the group consisting of a plasticizer, anti-caking agent, pigment, dye, substance preventing foaming of the coating suspension during the coating process, and mixtures thereof.

10. Enteric-coated duloxetine tablet according to claim 9, **characterized in that** one or more pharmaceutically acceptable excipients present in the enteric coating is a mixture of the plasticizer in the percentage of 8-12% by weight, preferably 10% by weight, the anti-caking agent in the percentage of the 18-22% by weight, preferably 20 % by weigh, and the pigment in the percentage of the 2-5% by weight, preferably 3% by weight, calculated on the total weight of the enteric coating.

11. Enteric-coated duloxetine tablet according to claim 10, **characterized in that** the plasticizer is triethyl citrate, the anti-caking agent is talc, and the pigment is titanium dioxide.

12. Enteric-coated duloxetine tablet according to any of the claims 1-11, **characterized in that** the percentage of the enteric coating is 8-13 % by weight, preferably 9-10 % by weight, calculated on the total weight of the enteric-coated tablet.

13. Enteric-coated duloxetine tablet according to any of the claims 1-12, **characterized in that** it comprises duloxetine hydrochloride in an amount equivalent to 20, 30, 40 or 60 mg of duloxetine in a form of a free base.

14. A process for the preparation of enteric-coated tablet according to claims 1-13, **characterized in that:** the process comprises the steps of:
a) preparing a blend of duloxetine hydrochloride with compressible sugar and optionally one or more pharmaceutically acceptable excipients, wherein the blend is prepared without the use of any liquid,
b) tableting the resulting blend to form tablet cores, and
c) coating the tablet cores with enteric coating material comprising methacrylic acid-ethyl acrylate copolymer (1:1) and one or more pharmaceutically acceptable excipients, wherein said coating is applied directly onto the tablet core.

15. The process according to claim 14, **characterized in that** in step a) duloxetine hydrochloride is blended with compressible sugar and a glidant, preferably corn starch, the resulting blend is subjected to dry granulation, then the resulting granulate is mixed with a lubricant, being magnesium stearate and thus obtained blend is subjected to tableting step b).

## Patentansprüche

1. Magensaftresistente Duloxetin-Tablette, bestehend aus:
- einem Kern aus Duloxetin-Hydrochlorid, welches ohne Verwendung einer Flüssigkeit gleichmäßig mit verpressbarem Zucker gemäß Definition in der Monographie des britischen Pharmacopoeas 2013, Band II, Seite 2117-2118, und gegebenenfalls mit einem oder mehreren pharmazeutisch geeigneten Hilfsstoffen gemischt wird, wobei die Menge des verpressbaren Zuckers bezogen auf das Gesamtgewicht des Kerns 60 bis 85 Gew.-% beträgt,
und
- einem magensaftresistenten Überzug, der direkt auf den Kern aufgebracht ist und aus Methacrylsäure-Ethylacrylat-Copolymer (1:1) als einzigem magensäureresistentem Polymer sowie einem oder mehreren pharmazeutisch geeigneten Hilfsstoffen besteht.

2. Magensaftresistente Duloxetin-Tablette nach Anspruch 1, **dadurch gekennzeichnet, dass** der verpressbare Zucker eine sprühgetrocknete Mischung aus Saccharose, Invertzucker und Maltodextrinen ist, wobei, bezogen auf das Gesamtgewicht des verpressbaren Zuckers, der Prozentsatz an Saccharose 95-98 Gew.-% und der Prozentsatz an Invertzucker und Maltodextrinen insgesamt 2-5 Gew.-% beträgt.

3. Magensaftresistente Duloxetin-Tablette nach Anspruch 1 oder 2, **dadurch gekennzeichnet, dass** der Prozentsatz des verpressbaren Zuckers bezogen auf das Gesamtgewicht des Kerns 65 bis 70 Gew.-%, vorzugsweise 67 Gew.-% beträgt.

4. Magensaftresistente Duloxetin-Tablette nach einem der Ansprüche 1 bis 3, **dadurch gekennzeichnet, dass** der Prozentsatz von Duloxetinhydrochlorid bezogen auf das Gesamtgewicht des Kerns 15 bis 30 Gew.-%, vorzugsweise 23 Gew.-% beträgt.

5. Magensaftresistente Duloxetin-Tablette nach einem der Ansprüche 1 bis 4, **dadurch gekennzeichnet, dass** ein oder mehrere pharmazeutisch geeignete Hilfsstoffe im Kern vorzugsweise aus der Gruppe bestehend aus einem Fließregulierungsmittel, Gleitmittel und Gemischen davon ausgewählt sind.

6. Magensaftresistente Duloxetin-Tablette nach Anspruch 5, **dadurch gekennzeichnet, dass** ein oder mehrere pharmazeutisch geeignete Hilfsstoffe im Kern eine Mischung aus dem Fließregulierungsmittel in der Menge von 7-11 Gew.-%, vorzugsweise 9 Gew.-%, und dem Gleitmittel in der Menge von 0,5 bis 3 Gew.-%, vorzugsweise zwischen 1 und 0,5 Gew.-%, am besten 0,5 Gew.-%, bezogen auf das Gesamtgewicht des Kerns, sind.

7. Magensaftresistente Duloxetin-Tablette nach Anspruch 6, **dadurch gekennzeichnet, dass** das Fließregulierungsmittel Maisstärke und das Gleitmittel Magnesiumstearat ist.

8. Magensaftresistente Duloxetin-Tablette nach einem der Ansprüche 1 bis 7, **dadurch gekennzeichnet, dass** der Prozentsatz von Methacrylsäure und Ethylacrylat-Copolymer (1:1) bezogen auf das Gesamtgewicht des magensaftresistenten Überzugs 60-70 Gew.-%, bevorzugt 67 Gew.-% beträgt.

9. Magensaftresistente Duloxetin-Tablette nach einem der Ansprüche 1 bis 8, **dadurch gekennzeichnet, dass** ein oder mehrere im magensaftresistenten Überzug enthaltene pharmazeutisch geeignete Hilfsstoffe aus der Gruppe bestehend aus einem Weichmacher, Formtrennmittel, Pigment, Farbstoff, einer Substanz, die das Schäumen der Überzugssuspension während des Ummantelungsvorgangs verhindert, und Mischungen davon ausgewählt sind.

10. Magensaftresistente Duloxetin-Tablette nach Anspruch 9, **dadurch gekennzeichnet, dass** ein oder mehrere im magensaftresistenten Überzug enthaltene pharmazeutisch geeignete Hilfsstoffe eine Mischung des Weichmachers in einem Prozentsatz von 8-12 Gew.-%, vorzugsweise 10 Gew.-%, des Formtrennmittels in einem Prozentsatz von 18-22 Gew.-%, vorzugsweise 20 Gew.-%, und Pigment in einem Prozentsatz von 2-5 Gew.-%, vorzugsweise 3 Gew.-%, bezogen auf das Gesamtgewicht des magensaftresistenten Überzugs, sind.

11. Magensaftresistente Duloxetin-Tablette nach Anspruch 10, **dadurch gekennzeichnet, dass** der Weichmacher Triethylcitrat ist, das Formtrennmittel Talkum ist und das Pigment Titandioxid ist.

12. Magensaftresistente Duloxetin-Tablette nach einem der Ansprüche 1 bis 11, **dadurch gekennzeichnet, dass** der Prozentsatz des magensaftresistenten Überzugs bezogen auf das Gesamtgewicht der magensaftresistenten Tablette 8 bis 13 Gew.-%, vorzugsweise 9 bis 10 Gew.-% beträgt.

13. Magensaftresistente Duloxetin-Tablette nach einem der Ansprüche 1 bis 12, **dadurch gekennzeichnet, dass** sie Duloxetinhydrochlorid in einer Menge enthält, die 20, 30, 40 oder 60 mg Duloxetin in Form der freien Base entspricht.

14. Verfahren zur Herstellung einer magensaftresistenten Duloxetin-Tablette nach den Ansprüchen 1 bis 13, **dadurch gekennzeichnet, dass** das Verfahren die folgenden Schritte umfasst:
a) Herstellen einer Mischung aus Duloxetinhydrochlorid und verpressbarem Zucker sowie einem oder mehreren pharmazeutisch geeigneten Hilfsstoffen, wobei die Mischung ohne die Verwendung irgendeiner Flüssigkeit hergestellt wird,
b) Tablettieren der resultierenden Mischung, um Tablettenkerne zu bilden, und
c) Ummantelung der Tablettenkerne mit magensäureresistentem Überzugsmaterial aus Methacrylsäure-Ethylacrylat-Copolymer (1:1) und einem oder mehreren pharmazeutisch geeigneten Hilfsstoffen, wobei der Überzug direkt auf den Tablettenkern aufgebracht wird.

15. Verfahren nach Anspruch 14, **dadurch gekennzeichnet, dass** in Schritt (a) Duloxetinhydrochlorid mit verpressbarem Zucker und einem Fließregulierungsmittel, vorzugsweise Maisstärke, gemischt wird, die resultierende Mischung einer Trockengranulierung unterzogen wird, dann das resultierende Granulat mit einem Gleitmittel, und zwar Magnesiumstearat, gemischt wird, und das so erhaltene Gemisch dem Tablettierungsschritt (b) unterzogen wird.

## Revendications

1. Comprimé de duloxétine à enrobage entérique constitué de :
- un noyau comprenant du chlorhydrate de duloxétine, qui est mélangé de manière uniforme sans utiliser de liquide avec du sucre compressible, tel que défini dans la monographie de la *British Pharmacopoeia* 2013, volume II, pages 2117 à 2118, la quantité dudit sucre compressible étant comprise entre 60 et 85 % en poids, calculée sur le poids total du noyau, et éventuellement avec un ou plusieurs excipients pharmaceutiquement acceptables, et
- un enrobage entérique appliqué directement sur le noyau et comprenant un copolymère d'acide méthacrylique et d'acrylate d'éthyle (1:1) en tant que seul polymère résistant à l'acide gastrique et un ou plusieurs excipients pharmaceutiquement acceptables.

2. Comprimé de duloxétine à enrobage entérique selon la revendication 1, **caractérisé en ce que** le sucre compressible est un mélange séché par atomisation de saccharose, de sucre inverti et de maltodextrines, dans lequel le pourcentage de saccharose est 95- 98 % en poids, et le pourcentage de sucre inverti et de maltodextrines au total de 2-5 % en poids, calculés par rapport au poids total du sucre compressible.

3. Comprimé de duloxétine à enrobage entérique selon la revendication 1 ou 2, **caractérisé en ce que** le pourcentage de sucre compressible est compris entre 65 et 70 % en poids, de préférence 67 % en poids, calculé sur le poids total du noyau.

4. Comprimé de duloxétine à enrobage entérique selon l'une quelconque des revendications 1 à 3, **caractérisé en ce que** le pourcentage de chlorhydrate de duloxétine est compris entre 15 et 30 % en poids, de préférence 23 % en poids, calculé sur le poids total du noyau.

5. Comprimé de duloxétine à enrobage entérique selon l'une quelconque des revendications 1 à 4, **caractérisé en ce qu'**un ou plusieurs excipients pharmaceutiquement acceptables dans le noyau sont de préférence choisis dans le groupe constitué d'un agent d'écoulement, d'un lubrifiant et de leurs mélanges.

6. Comprimé de duloxétine à enrobage entérique selon la revendication 5, **caractérisé en ce qu'**un ou plusieurs excipients pharmaceutiquement acceptables dans le noyau sont un mélange d'agent glissant en une quantité de 7-11 % en poids, de préférence 9 % en poids et de lubrifiant en une quantité de 0,5-3 % en poids, de préférence de 1-0,5 % en poids, le plus préférablement 0,5 % en poids, calculée sur le poids total du noyau.

7. Comprimé de duloxétine à enrobage entérique selon la revendication 6, **caractérisé en ce que** l'agent d'écoulement est l'amidon de maïs et le lubrifiant est le stéarate de magnésium.

8. Comprimé de duloxétine à enrobage entérique selon l'une quelconque des revendications 1 à 7, **caractérisé en ce que** le pourcentage de copolymère d'acide méthacrylique et d'acrylate d'éthyle (1:1) est compris entre 60 et 70 % en poids, plus préférablement 67 % en poids, calculé sur le poids total du revêtement entérique.

9. Comprimé de duloxétine à enrobage entérique selon l'une quelconque des revendications 1 à 8, **caractérisé en ce qu'**un ou plusieurs excipients pharmaceutiquement acceptables présents dans l'enrobage entérique sont choisis dans le groupe constitué par un plastifiant, un anti-agglomérant, un pigment, un colorant, une substance empêchant le moussage de la suspension d'enrobage pendant le procédé d'enrobage, et leurs mélanges.

10. Comprimé de duloxétine à enrobage entérique selon la revendication 9, **caractérisé en ce qu'**un ou plusieurs excipients pharmaceutiquement acceptables présents dans l'enrobage entérique sont un mélange de plastifiant en pourcentage de 8-12 % en poids, de préférence 10% en poids, d'anti-agglomérant en pourcentage de 18-22 % en poids, de préférence 20 % en poids, et de pigment en pourcentage de 2-5 % en poids, de préférence 3 % en poids, calculé sur le poids total de l'enrobage entérique.

11. Comprimé de duloxétine à enrobage entérique selon la revendication 10, **caractérisé en ce que** le plastifiant est le citrate de triéthyle, l'anti-agglomérant est le talc et le pigment est le dioxyde de titane.

12. Comprimé de duloxétine à enrobage entérique selon l'une quelconque des revendications 1 à 11, **caractérisé en ce que** le pourcentage de l'enrobage entérique est compris entre 8-13 % en poids, de préférence de 9-10 % en poids, calculé sur le poids total du comprimé à enrobage entérique.

13. Comprimé de duloxétine à enrobage entérique selon l'une quelconque des revendications 1 à 12, **caractérisé en ce qu'**il comprend du chlorhydrate de duloxétine en une quantité équivalente à 20, 30, 40 ou 60 mg de duloxétine sous forme de base libre.

14. Procédé de préparation d'un comprimé à enrobage entérique selon les revendications 1 à 13, **caractérisé en ce que** le procédé comprend les étapes de :
a) préparation d'un mélange de chlorhydrate de duloxétine avec du sucre compressible et un ou plusieurs excipients pharmaceutiquement acceptables, dans lequel le mélange est préparé sans utiliser de liquide,
b) compression du mélange résultant pour former les noyaux des comprimés, et
c) enrobage des noyaux des comprimés avec un matériau d'enrobage entérique comprenant un copolymère d'acide méthacrylique et d'acrylate d'éthyle (1:1) et un ou plusieurs excipients pharmaceutiquement acceptables, ledit enrobage étant appliqué directement sur le noyau du comprimé.

15. Procédé selon la revendication 14, **caractérisé en ce que,** dans l'étape a), le chlorhydrate de duloxétine est mélangé avec du sucre compressible et un agent d'écoulement, de préférence de l'amidon de maïs, le mélange résultant est soumis à une granulation à sec, puis le granulat résultant est mélangé avec un lubrifiant, à savoir le stéarate de magnésium et le mélange ainsi obtenu est soumis à l'étape de compression en b).
